# EUROPEAN PATENT SPECIFICATION

(11) **EP 1 768 646 B1**
(45) Date of publication and mention of the grant of the patent: **09.05.2018**
(21) Application number: 05747830.7
(22) Date of filing: 30.05.2005
(51) Int. Cl.: A61Q 15/00, A61K 8/33, A61K 8/34, A61K 8/04

(54) **COSMETIC SPRAYS**
KOSMETIKSPRAYS
SPRAYS COSMÉTIQUES

(30) Priority: 17.06.2004 GB 0413582; 15.12.2004 GB 0427447
(43) Date of publication of application: 04.04.2007
(73) Proprietor: Unilever PLC, London EC4Y 0DY (GB); Unilever N.V., 3013 AL Rotterdam (NL)
(72) Inventor: COXON, Andrew, Charles, Bromborough, Merseyside CH62 6EP (GB); SHEARMUR, T., Unilever R & D Port Sunlight, Bebington Merseyside CH63 3JW (GB); KUTAY, Susan, Michelle, Port Moody, British Columbia V3H 5K5 (CA)
(74) Representative: Whaley, Christopher
(86) International application number: PCT/EP2005/005853
(87) International publication number: WO 2005/123025

(56) References cited:
- WO-A2-96/18378
- DE-A1- 19 856 567
- US-A- 3 544 258
- US-A- 5 221 531
- US-A1- 2001 051 135
- US-B1- 6 689 340
- US-B2- 6 653 353
- WARREN LITSKY ET AL: "STERILITY TESTING AND ANTIMICROBIAL ACTIVITY OF COMMERCTAL GRADE GLYCERINE", INTERNET CITATION, 1 August 1971 (1971-08-01), pages 1-27, XP007919273, Retrieved from the Internet: URL:http://www.aciscience.org/docs/Sterili ty_Testing_and_Antimicrobial_Activity_of_C ommercial_Grade_Glycerine.pdf [retrieved on 2011-08-24]

## Description

### Field of Invention

This invention relates to cosmetic spray compositions having a low VOC (volatile organic carbon) content; in particular, it relates to cosmetic compositions that deliver a deodorancy benefit.

### Background

A large number of cosmetic compositions suitable for spray application to the human body are known. Good quality spray generation has been found possible using cosmetic compositions comprising high levels of volatile propellant and/or ethanol. However, as concerns over the effects of VOCs upon to environment increase, so does the desire to use compositions comprising less of such components. Water is an excellent, environmentally acceptable alternative to ethanol; however, water is not miscible with many of the volatile propellants commonly used and this can lead to formulation problems.

A solution to the above-mentioned miscibility problem is the use of dimethylether (DME) as the volatile propellant, in combination with ethanol. The three component system water-DME-ethanol is monophasic over a wide range of formulation space. The use of such systems is described in US 3,544,258 (F. Presant et al, 1970); JP 63/137,981 (Kanebo, 1988); JP 02/032,190 (Osaka Aerosol Kogyo, 1990); GB 1,300,399 (Aerosol Maatschappij Holland, 1972); and DE 3,347,742 (IG Spruchtechnik, 1985). None of the above publications, however, disclose the selected area of water-DME-ethanol formulation space that is key to the attainment of the benefits deriving from the present invention.

### Summary of Invention

It is the main object of the present invention to provide a low VOC cosmetic spray composition that gives good spray quality. The route adopted is *via* optimisation of DME-water-ethanol compositions. Since DME and ethanol are both VOCs, these components are minimised in the compositions of the invention and the water content is maximised. However, certain amounts of DME and ethanol are required in order for the compositions of the invention to have the physical properties (in particular pressure and, to a lesser extent, surface tension) that lead towards good spray quality and good sensory perception for a spray generated therefrom. It is also important that the ratio of DME to ethanol be kept within closely defined parameters in order to achieve the desired physical parameters. Further, it is important that a certain amount of ethanol is maintained in the compositions in order to ease the incorporation of additional organic components, such as fragrance materials. These sometimes-competing requirements are all met by the present invention.

According to a first aspect of the present invention, there is provided a cosmetic spray composition according to claim 1. According to a second aspect of the present invention, there is provided a consumer product comprising a cosmetic spray composition according to the first aspect of the invention and a device for producing a spray therefrom.

According to a third aspect of the present invention, there is provided a method of cosmetic treatment comprising the spraying of a composition according to the first aspect of the invention onto the surface of the human skin.

It is a further object of the present invention to provide a cosmetic composition, suitable for application to the surface of the human body, which has good sensory properties.

The cosmetic composition according to the first aspect of the invention involves ethanol, DME and water, in the specified amounts and specified weight ratio of DME to ethanol, as a carrier fluid for a cosmetic composition comprising an additional cosmetic active. For the many cosmetic actives that are organic, the presence of ethanol in the composition greatly eases their incorporation therein.

It is a particular object of the present invention to provide a low VOC deodorant spray composition having a good spray quality. One of the means that has been found for delivering the deodorancy benefit is the inclusion in compositions of the invention of two additional cosmetic deodorant actives including a fragrance and an anti-microbial agent. The use of an anti-microbial agent is particularly desirable because it can dramatically increase deodorancy performance without increasing the VOC content of the composition.

### Detailed Description

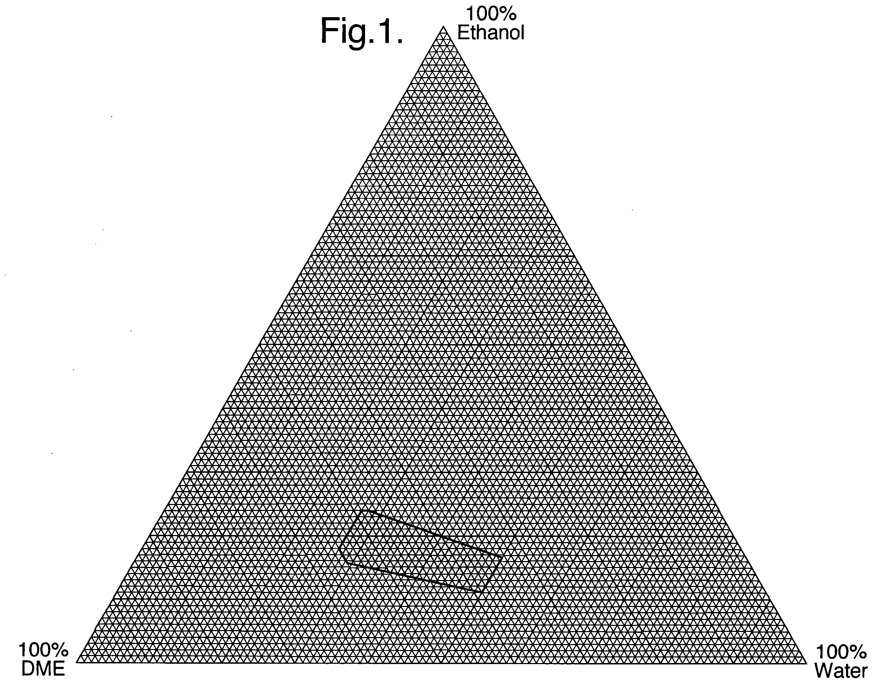

The quantitative requirements of a cosmetic composition according to the first aspect of the invention may be represented on an ethanol-DME-water three-component phase diagram. Figure 1 is such a phase diagram where the shaded area represents the 'formulation space' of cosmetic compositions according to the first aspect of the invention. Compositions falling within this relatively small area of formulation space give the benefits that are the aim of the present invention. Three essential components of the compositions of the invention (i.e. ethanol, DME, and water) represent at least 95% by weight of the total composition. These high levels improve the attainment of the benefits of the invention, in particular the benefit of good spray quality. As the level of 'other components' increases, the benefits of the invention can decrease.

Ethanol is present at from 11% to 24%, DME is present at from 33% to 55%, all percentages being by weight, based upon the total weight of ethanol, DME, and water present in the composition. The weight ratio of DME to ethanol in the composition is also essential to the functioning of the invention; this ratio is from 2:1 to 3.5:1, preferably from 2.5:1 to 3.5:1, and most preferably from 2.5:1 to 3.0:1. The above levels and ratios of components enable a good quality spray having good sensory properties to be generated, despite the presence of considerable water in the composition.

The required minimum level of ethanol, and the required and preferred minimum ratios of ethanol to DME, also give benefits in aiding the incorporation of additional organic cosmetic actives, in particular fragrance (*vide infra*). These benefits result from the improved solubility of the additional organic cosmetic active in the ethanol, water, DME "carrier fluid". The improved solubility may lead to such benefits as reduced valve blockage, more uniform dispensing of the total composition, and avoidance of the need to shake the formulation before spraying.

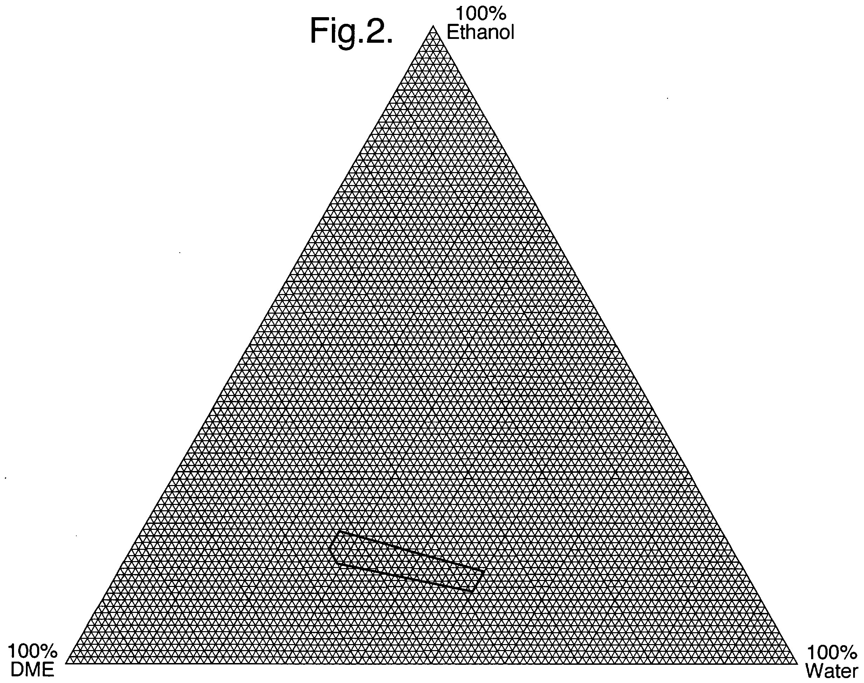

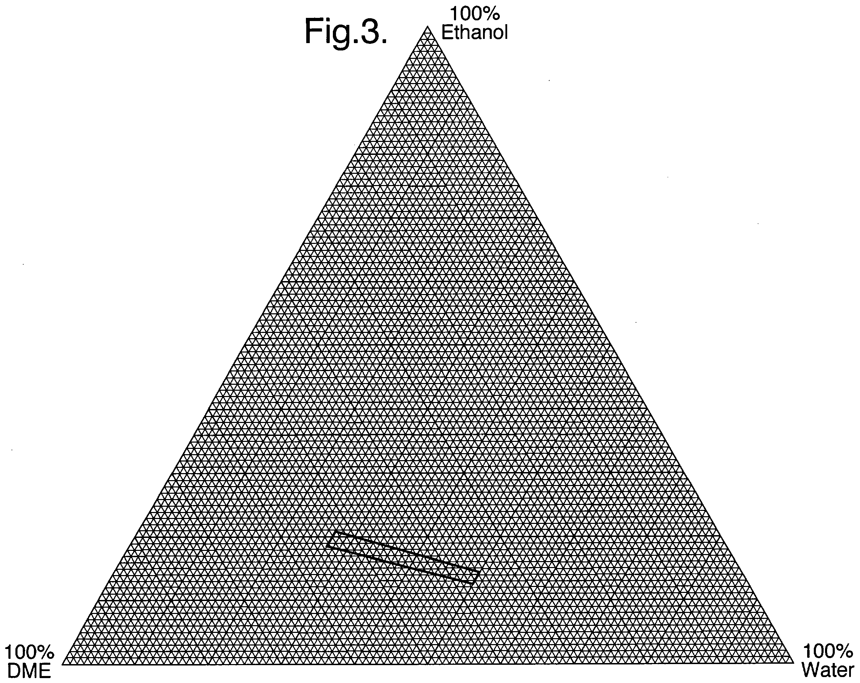

The formulation space covered when the weight ratio of DME to ethanol is from 2.5:1 to 3.5:1 is represented by the shaded area in Figure 2 and the formulation space covered when the weight ratio of DME to ethanol is from 2.5:1 to 3.0:1 is represented by the shaded area in Figure 3.

In order to attain the benefit of good spray quality, it is of help if the composition is capable of generating a pressure of from 43 to 55 psig (from 398 to 487 kPa) at a temperature of 21°C. A particularly helpful pressure for the composition to be capable of generating is from 48 to 55 psig (from 432 to 487 kPa) at a temperature of 21°C. The pressure generated by a composition may be measured by filling a standard aerosol can with the composition to a typical level, say 71% by volume, spraying off 50% by weight of the composition, and then measuring the can pressure using a pressure gauge.

In order for compositions to be perceived as having a good spray quality and good sensory properties it is of help for them to generate a droplet size that is neither too great nor too small. Preferred compositions are able to give a D[4,3] droplet size of from 10 to 50 microns and especially from 15 to 40 microns, when sprayed a temperature of 21°C using a conventional dispenser.

Droplet size measurements referred to in this description are made 15 cm from the spray orifice, typically using light scattering techniques, typically with instruments such as the Malvern Mastersizer.

The cosmetic compositions of the present invention comprise at least two additional cosmetic actives, and the ethanol, DME, and water may be considered as a carrier fluid for the additional cosmetic actives. It is preferred that the total amount of additional cosmetic actives is not too great, in order not to interfere with the good spray quality and sensory benefits delivered by the carrier fluid. The total amount of additional cosmetic actives is 5% or less by weight of the total composition.

In order to serve its purpose, it is important that the additional cosmetic active be present in a sufficient amount. It is preferred that a particular additional cosmetic active is present in an amount of from 0.1%, and preferably from 0.3% by weight of the total composition.

The additional cosmetic active may be selected from any of those known in the art. Particular classes of active include deodorants; skin benefit agents, including sunscreens, desquamating agents, emollients, humectants, anti-ageing agents, wrinkle-reducing agents, skin whitening agents, sebum reducing agents, and skin moisturisers; hair growth modifiers or eliminators (i.e. depilatories); astringents; cooling agents; and colorants.

Additional cosmetic actives that are organic are preferred, as such components tend to be compatible with the carrier fluid, particularly the ethanol therein. It is especially preferred that the additional cosmetic active is soluble in the ethanol-DME-water carrier fluid.

Deodorant actives, including fragrances and anti-microbial agents, are essential ingredients according to the present invention and skin benefit agents, in particular skin moisturisers, are additional cosmetic actives that are particularly suitable for use with the present invention.

Organic deodorant actives are especially suitable for use with the present invention. This is in part due to their physical compatibility with the carrier fluid, enabling good quality spray generation, and in part due to the possibility of the carrier fluid augmenting the deodorancy performance of the organic deodorant active. In addition, the particular ratio of DME to ethanol in the compositions of the invention aids good solubilisation of organic deodorant actives, in particular of fragrances (*vide infra*). An essential additional organic deodorant active is a fragrance. The term 'fragrance' should be considered synonymous with 'perfume' and includes all components of a multi-component fragrance when such is present. As a fragrance is present, the co-presence of the required proportion of ethanol in the composition is particularly important in order to aid the fragrance's solubility in the composition. The fragrance may comprise simple volatile odoriferous molecules and/or may comprise so-called "deo-perfume" molecules that have anti-microbial properties. A fragrance is present in an amount of from 0.1 in particular 0.2 to 3%, and especially 0.5 to 2% by weight of the total composition, these amounts referring to the total weight of fragrance components present.

Deodorant actives may work by absorbing malodour or by preventing malodour formation. Deodorant actives functioning by the prevention of the formation of malodour are particularly preferred. (In this description, "preventing" includes partially preventing, i.e. reducing). Such actives may function by preventing the formation of sweat (i.e. as antiperspirants) and/or by preventing the microbial breakdown of sweat components into odorous molecules. Indeed, essential deodorant actives are anti-microbial agents and function by this latter mechanism.

Deodorant actives that are anti-microbial agents are preferably organic and may be bactericides or bacteriostats. It should be noted that whilst ethanol can be a deodorant active, it is not an 'additional cosmetic active' as herein described because it is not an 'additional' component, being present as part of the ethanol-DME-water 'carrier fluid'. Suitable deodorant actives include: quaternary ammonium compounds, such as cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). Preferred anti-microbial agents are polyalkylene biguanide salts; poly(alkylene-guanidine) salts; poly(oxyalkylene-guanidine) salts; zinc phenol sulphonate; 2',4,4'-trichloro,2-hydroxydiphenyl ether (triclosan); and 3,7,11-trimethyldodeca-2,6,10-trienol (farnesol). Particularly preferred anti-microbial agents are polyalkylene biguanide salts; poly(alkylene-guanidine) salts, for example poly(hexamethylene-guanidine hydrochloride); poly(oxyalkylene-guanidine) salts, for example poly(triethylene-glycol-guanidine hydrochloride); and zinc phenol sulphonate. The above particularly preferred anti-microbial agents are highly compatible with (i.e. soluble in) the ethanol-DME-water carrier fluid, giving rise to superior compositions. Polyhexamethylene biguanide salts, for example Cosmocil CQ available from Arch Chemicals Inc., are especially preferred anti-microbial agents.

In some embodiments of the invention, organic anti-microbial agents that are bacteriostats are used. Suitable bacteriostats are iron(III) chelators, in particular those having an iron(III)-binding coefficient of 10²⁶ or greater. Preferred organic bacteriostatic iron (III) chelators are aminopolycarboxylic acids and salts thereof, in particular the following acids and their salts:
diethylenetriaminepentaacetic acid (DTPA), triethylenetetraaminehexaacetic acid (TTHA), and N,N'-ethylenebis[2-(2-hydroxyphenyl)glycine] (EDDHA).

Organic bacteriostatic iron(III) chelators are preferably used in combination with an organic anti-microbial agent that is a phenolic or enolic compound as described in the following paragraph.

The composition may comprise an organic anti-microbial agent that is a phenolic or enolic compound that is (a) a transferrin dissociation promoter that operates by aiding the reduction of iron(III) bound to transferrin to iron(II) and/or (b) an anti-oxidant comprising a *tert*-butylphenol group. Such materials are described in WO 02/30383. Preferably, such materials are selected from ascorbic acid (and salts thereof), ascorbyl-6-palmitate, butylated hydroxytoluene, 2,2'-ethylidenebis(4,6-di-*tert-*butylphenol), or pentaerythritol tetrakis(3-(3,5-di-*tert-*butyl-4-hydroxyphenyl)propionate). Butylated hydroxytoluene is especially preferred.

The total amount of organic anti-microbial agent, when present, is from 0.1, and preferably from 0.2 to 2% of the of the total composition.

In order to enhance the sensory benefits of sprays generated from compositions according to the invention, a humectant, such as a polyol, may be employed. Preferred humectants are glycerol, propylene glycol, and dipropylene glycol; propylene glycol is particularly preferred.

The sensory benefits of sprays generated from compositions according to the invention, may be enhanced by the presence of an emollient. Such materials are typically oils and are emulsified into an aqueous continuous phase. Suitable oils include silicone oils, such as DC244, DC245, DC344 and DC345, ex Dow Corning; alkyl esters, such as PurSyn Ester 2E7 (neopentylglycol dihexanoate), ex Exxon-Mobil; benzoate esters such as Finsolv TN (Trade Mark), ex Finetex Inc.; hydrogenated polyalkenes, such as Panalane, ex Amoco and PureSyn PAO 2 (hydrogenated polydecene), ex Exxon-Mobil; PPG ethers, such as Fluid AP (PPG-14 butylether), ex Union Carbide; isopropyl palmitate; phenylsilicone; and isopropyl myristate.

Particularly preferred compositions according to the present invention have a polyalkyleneoxide polysiloxane surfactant present to aid their sensory perception. Such materials can reduce the perception of wetness that may result from the presence of water in the compositions of the invention.

Preferred polyalkyleneoxide polysiloxane surfactants are polyalkyleneoxide-modified polydimethylsiloxanes. These latter materials may be of general formula I: 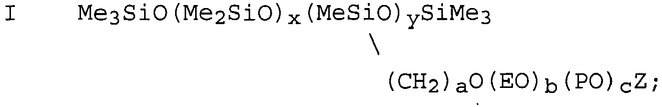 where Me represents methyl; EO represents ethyleneoxy; PO represents 1,2-propyleneoxy; , x is from 0 to 10, preferably from 0 to 5, and most preferably 0; y is from 1 to 8, preferably from 1 to 3, and most preferably 1; a is from 2 to 4, preferably 3; b is from 1 to 15, preferably from 5 to 15; c is from 0 to 14, preferably from 0 to 3; b + c is from 5 to 15, preferably from 6 to 10; and Z can be either hydrogen or a lower alkyl group.

For optimum performance, preferred polyalkyleneoxide polysiloxane surfactants have a molecular weight (MW) of less than 1000, in particular less than 800, and especially less than 700. In polyalkyleneoxide polysiloxane surfactants of formula I, the number of EO units must be sufficient to render the surfactant dispersible or soluble in the composition/spray. When c is non-zero in formula I, the EO and PO units may be distributed randomly, in respective blocks of EO and PO units, or in a combination of random and block distributions.

Preferred polyalkyleneoxide polysiloxane surfactants have an estimated HLB value of from 5-8, the HLB value of the surfactant being estimated from its aqueous solubility and cloud point using the method described by Griffin (Off. Dig. Paint and Varnish Productions Clubs, 28, 446, 1956) and later by Schott (J. Pharm. Sci., 58, 1442, 1969).

Suitable polyalkyleneoxide polysiloxane surfactants that are commercially available may be selected from Silwet L-7280, Silwet L-77, and Silwet L-7608, all available from OSi, and DC Q2-5211, available from Dow Corning Corp.

When present, the polyalkyleneoxide polysiloxane surfactant is typically used at a level from 0.1%, particularly at from 0.1 to 2.5%, and especially at from 0.1 to 1% by weight of the total composition. Especially preferred compositions comprise fragrance at a level of from 1% by weight and polyalkyleneoxide polysiloxane surfactant at a level of from 0.3% by weight of the total composition.

The polyalkyleneoxide polysiloxane surfactant may also function as a perfume solubiliser, as may other nonionic surfactants such as fatty alcohol ethoxylates or PEG esters or ethers of fatty acids or glycerides. In compositions of the invention a perfume solubiliser is a preferred additional component. In such compositions, the perfume solubiliser may help to minimise the amount of ethanol required in the formulation for perfume solubilisation and thereby reduce the total VOC content of the composition. Preferred perfume solubilisers are PEG derivatives of a triglyceride, in particular a PEG ether of an hydroxylated triglyceride, such as PEG-60 hydrogenated castor oil. The total amount of perfume solubiliser in the composition of the invention is preferably from 0.1% to 3% by weight of the total composition. In general, the weight ratio of the total amount of perfume solubiliser to the total amount of perfume (fragrance) is preferably from 1:4 to 1:1 and more preferably from 1:3 to 1:1.

Other components that may be present include inorganic electrolytes, such as sodium chloride or sodium sulphate; rheology modifiers, such as hydroxypropyl celluloses; silicone gum, such as Q2 1501, ex Dow Corning; colourants; and preservatives, such as Glydant or parabens.

Spray devices suitable for use with the compositions of the invention typically comprise a pressure canister, a valve, and a nozzle. Such devices are well known in the art. In order to aid the spray quality, it is preferred that the spray device comprises a means for enhancing the atomisation of the spray, for example a mechanical break up unit such as a swirl chamber. Such means can help in attaining the droplet size distributions desired from spray compositions according to the invention.

It is greatly preferred that the pressure canister is made from metal, for example tinplate or, more preferably, aluminium. Canisters made from metal have the benefits of being better able to withstand the pressure generated by the composition and being less prone to weakening ingress of components of the composition into the canister material. Problems regarding the aforementioned issues can arise with canisters made of other materials, for example plastic.

Metal canisters are preferably lacquered on their inside, for example with an epoxyphenolic resin or, more preferably, with polyamide imide resin. The lacquer serves to maintain the ability of the can to withstand the pressure generated by the composition, an ability that can be compromised by corrosion of the canister by the water-containing compositions of the present invention.

It is preferred that the spray device is capable of containing and dispensing a cosmetic composition held at a pressure of from 43 to 55 psig (from 398 to 487 kPa) at 21°C, in particular at from 48 to 55 psig (from 432 to 487 kPa) at 21°C.

It is highly preferred that the spray device is capable of generating a spray of D[4,3] droplet size of from 10 to 50 microns and especially of from 15 to 40 microns when a composition according to the invention is sprayed at 21°C. The canisters are typically filled to a level of from 62% to 77% by volume with the composition. In general, the components of the composition other than the DME are first added, followed by the DME under sufficient pressure to keep it liquefied at the operating temperature (typically 21°C).

### Examples

In the following examples, comparative examples are indicated by letters and examples according to the invention are indicated by numbers and "invention" label. All tabulated amounts are percentages by weight of the total composition.

The examples indicated in Table 1 were prepared by standard methods in the art. The compositions were contained in and dispensed from standard (210 ml) aluminium aerosol dispensers equipped a swirl chamber nozzle. 150 ml of indicated compositions were placed in the aerosol dispensers. Table 1 indicates compositional details for the examples; the can pressures obtained (after 50% by weight of the composition had been discharged); and the D[4,3] droplet sizes obtained upon spraying.

**Table 1: Compositions, pressures, and droplet sizes**

| **Example** | **EtOH (%)** | **DME (%)** | **Water (%)** | **Can pressure (psig [kPa])*** | **D [4,3] (microns)*** |
|---|---|---|---|---|---|
| **A** | 25 | 50 | 25 | 50 [446] | 20 |
| **B** | 30 | 40 | 30 | 40 [377] | 39 |
| **1** | 20 | 50 | 30 | 48 [432] | 27 |
| **2** | 20 | 40 | 40 | 43 [398] | 35 |
| **3** | 15 | 45 | 40 | 49 [439] | 22 |
| **D** | 20 | 30 | 50 | 39 [370] | >100 |
| **4** | 12.5 | 37.5 | 50 | 48 [432] | 36 |

| | | | | | |
|---|---|---|---|---|---|
| * Measured at 21°C. | | | | | |

Comparative Example A gives an acceptable can pressure and droplet size; however, it contains too much VOC (ethanol and DME) to deliver the main object of the present invention.

Comparative Example B comprises too much ethanol, particularly when compared with the amount of DME present. As a result, it does not achieve the desired can pressure.

Example 1 has a DME to ethanol weight ratio of 2.5:1 and gives a good can pressure of 48 psig (432 kPa). Similarly, Examples 3 and 4, both of which have a DME to ethanol weight ratio of 3:1, give good can pressures of 49 psig (439 kPa) and 48 psig (432 kPa) respectively. Example 2, which has a DME to ethanol weight ratio of 2:1, gives an acceptable can pressure of 43 psig (398 kPa).

Comparative Example D comprises too little DME, particularly when compared with the amount of ethanol present. As a result, it does not achieve the desired can pressure and nor does it give the desired D[4,3] droplet size.

The examples indicated in Table 2 were prepared by standard methods in the art. Each of these compositions comprised a perfume and a perfume solubiliser (Cremophor CO60). Example has a DME to ethanol ratio of 3:1. This example has a clear appearance. Comparative example E is just outside the scope of the invention, having a DME to ethanol ratio of 4:1. This sample is cloudy, indicating a lack of complete solubilisation of the perfume, despite the perfume and perfume solubiliser being at the same levels as in Example 5.

**Table 2: Perfumed Compositions and Appearance**

| **Components** | **Example** | | | |
|---|---|---|---|---|
| | **5** | **E** | **6** | **7** |
| DME | 37.5 | 40.0 | 37.5 | 37.5 |
| Ethanol | 12.5 | 10.0 | 12.5 | 12.5 |
| Water | 47.4 | 47.4 | 47.0 | 48.2 |
| Perfume | 1.5 | 1.5 | 1.5 | 1.5 |
| Cremophor CO60 | 1.1 | 1.1 | 1.5 | 0.3 |
| **Appearance:** | Clear | Cloudy | Clear | Cloudy |

Example 6 has a perfume solubiliser to perfume ratio of 1:1 and Example 7 has a perfume solubiliser to perfume ratio of 1:5, this latter ratio not being ideal for solubilisation of the perfume in this particular formulation.

The examples indicated in Table 3 were prepared by standard methods in the art. Table 3 indicates compositional details for the examples and also their 'malodour scores', as assessed by expert assessors 5 and 24 hours after spray application for two seconds of the indicated example (low values indicate low malodour).

**Table 3: Compositions and Malodour Performance**

| **Component** | **Example** | | | |
|---|---|---|---|---|
| | **4** | **8** | **9** | **10** |
| Ethanol | 12.5 | 12.5 | 12.5 | 12.5 |
| DME | 37.5 | 37.5 | 37.5 | 37.5 |
| Water | 50 | 49 | 48.5 | 46 |
| AMP-95* | - | 0.4 | 0.4 | 0.4 |
| DTPA | - | 0.5 | 0.5 | 0.5 |
| BHT | - | 0.1 | 0.1 | 0.1 |
| Farnesol | - | - | 0.5 | - |
| Triethyl citrate | - | - | - | 3 |

| **Malodour score** | | | | |
|---|---|---|---|---|
| After 5 hr. | 2.49 | 1.97 | 1.74 | 2.00 |
| After 24 hr. | 2.96 | 2.01 | 2.09 | 2.11 |

| | | | | |
|---|---|---|---|---|
| * 2-amino-2-methyl-1-propanol (95% w/w) and water (5% w/w) | | | | |

Examples 8, 9, and 10 gave significantly lower malodour scores (at the 99% level) than Example 4, after both 5 hours and 24 hours. Examples 9 and 10 are "invention" examples. The further examples indicated in Table 4 were also prepared by standard methods in the art. In addition to a fragrance and anti-microbial agents, Example 11 also comprises a perfume solubiliser (Cremophor CO60) and Example 13 comprises a polyalkyleneoxide polysiloxane surfactant (Silwet L7608), to enhance the sensory properties, both examples being "invention" examples.

**Table 4: Further Compositions**

| **Component** | **Example** | | | |
|---|---|---|---|---|
| | **11** | **13** | **14** | **15** |
| Ethanol | 12.5 | 12.5 | 12.5 | 12.5 |
| DME | 37.5 | 37.5 | 37.5 | 37.5 |
| Water | 47.1 | 47.1 | 49.5 | 46.8 |
| AMP-95* | 0.4 | 0.4 | - | - |
| DTPA | 0.5 | 0.5 | - | - |
| BHT | 0.1 | 0.1 | - | - |
| Silwet L7608 | - | 0.5 | - | 0.5 |
| Cremophor CO60¹ | 0.5 | - | - | - |
| Cosmocil CQ² | - | - | 0.5 | - |
| ZPS³ | - | - | - | 2.7 |
| Fragrance | 1.4 | 1.4 | - | - |

| | | | | |
|---|---|---|---|---|
| * 2-ammo-2-methyl-1-propanol (95% w/w) and water (5% w/w) 1. PEG-60 hydrogenated castor oil, ex BASF. 2. Polyhexamethylene biguanide hydrochloride, ex Arch Chemicals Inc., a 20% w/w aqueous solution - hence 0.1% of anti-microbial agent was present in the final composition. 3. Zinc phenol sulphonate. | | | | |

## Claims

1. A cosmetic spray composition comprising ethanol, DME, and water in proportions of from 11% to 24% by weight ethanol, from 33% to 55% by weight DME, and from 27% to 50% by weight water based upon the total weight of ethanol, DME, and water present in the composition, the weight ratio of DME to ethanol being from 2:1 to 3.5:1, **characterised in that** it comprises two or more additional deodorant actives including at least one fragrance and at least one anti-microbial agent and **in that** a particular additional cosmetic active is present in an amount of from 0.1% and the total amount of additional cosmetic actives is 5% or less and **in that** the ethanol, DME, and water represent at least 95%, all percentages being by weight of the total composition.

2. A cosmetic spray composition according to claim 1, wherein the weight ratio of DME to ethanol is from 2.5:1 to 3.5:1.

3. A cosmetic spray composition according to any of the preceding claims that is able to generate a pressure of from 43 to 55 psig (398 to 487 kPa) at 21°C.

4. A consumer product comprising a cosmetic spray composition according to any of the preceding claims and a device for producing a spray therefrom.

5. A consumer product according to claim 4, wherein the spray device is capable of containing and dispensing a composition held at a pressure of from 43 to 55 psig (from 398 to 487 kPa) at 21°C.

6. A consumer product according to claim 4 or 5, wherein the spray device is capable of generating a spray of D[4,3] droplet size of from 10 to 50 microns when used to spray the composition at a temperature of 21°C.

7. A method of cosmetic treatment comprising the spraying of a cosmetic composition according to any of claims 1 to 3 onto the surface of the human skin.

## Patentansprüche

1. Kosmetische Sprühzusammensetzung, umfassend Ethanol, DME und Wasser in Anteilen von 11 bis 24 Gewichts-% Ethanol, von 33 bis 55 Gewichts-% DME und von 27 bis 50 Gewichts-% Wasser, bezogen auf das Gesamtgewicht von Ethanol, DME und Wasser, die in der Zusammensetzung vorliegen, wobei das Gewichtsverhältnis von DME zu Ethanol von 2:1 bis 3,5:1 beträgt, **dadurch gekennzeichnet, dass** sie zwei oder mehr zusätzliche Deodorant-Wirkstoffe enthält, einschließlich mindestens eines Duftstoffes und mindestens eines antimikrobiellen Mittels, und dass ein besonderer zusätzlicher kosmetischer Wirkstoff in einer Menge ab 0,1% vorliegt und die Gesamtmenge der zusätzlichen kosmetischen Wirkstoffe 5% oder weniger beträgt und dass das Ethanol, DME und Wasser mindestens 95% darstellen, wobei sich alle Prozentangaben auf das Gewicht der gesamten Zusammensetzung beziehen.

2. Kosmetische Sprühzusammensetzung nach Anspruch 1, wobei das Gewichtsverhältnis von DME zu Ethanol von 2,5:1 bis 3,5:1 beträgt.

3. Kosmetische Sprühzusammensetzung nach irgendeinem der vorhergehenden Ansprüche, die im Stande ist, bei 21°C einen Druck von 43 bis 55 psig (398 bis 487 kPa) zu erzeugen.

4. Verbraucherprodukt, umfassend eine kosmetische Sprühzusammensetzung nach irgendeinem der vorhergehenden Ansprüche und eine Vorrichtung, die damit einen Sprühstrahl erzeugt.

5. Verbraucherprodukt nach Anspruch 4, wobei die Sprühvorrichtung im Stande ist, eine Zusammensetzung, die bei 21°C unter einem Druck von 43 bis 55 psig (von 398 bis 487 kPa) gehalten wird, aufzubewahren und abzugeben.

6. Verbraucherprodukt nach Anspruch 4 oder 5, wobei die Sprühvorrichtung im Stande ist, einen Sprühstrahl von D[4,3] Tröpfchengröße von 10 bis 50 Mikrometer zu erzeugen, wenn sie verwendet wird, um die Zusammensetzung bei einer Temperatur von 21°C zu versprühen.

7. Kosmetisches Behandlungsverfahren, umfassend das Sprühen einer kosmetischen Zusammensetzung nach einem der Ansprüche 1 bis 3 auf die Oberfläche menschlicher Haut.

## Revendications

1. Composition de pulvérisation cosmétique comprenant de l'éthanol, du DME, et de l'eau dans des proportions de 11 % à 24 % en masse d'éthanol, de 33 % à 55 % en masse de DME, et de 27 % à 50 % en masse d'eau rapporté à la masse totale d'éthanol, de DME, et d'eau présents dans la composition, le rapport massique de DME à éthanol étant de 2:1 à 3,5:1, **caractérisée en ce qu'**elle comprend deux ou plusieurs matières actives de déodorants supplémentaires incluant au moins un parfum et au moins un agent anti-microbien et **en ce qu'**une matière active cosmétique supplémentaire particulière est présente dans une quantité de 0,1 % et la quantité totale de matières actives cosmétiques supplémentaires est de 5 % ou inférieure et **en ce que** l'éthanol, le DME, et l'eau représentent au moins 95 %, tous les pourcentages étant en masse de la composition totale.

2. Composition de pulvérisation cosmétique selon la revendication 1, dans laquelle le rapport massique de DME à éthanol est de 2,5:1 à 3,5:1.

3. Composition de pulvérisation cosmétique selon l'une quelconque des revendications précédentes qui est capable de produire une pression de 43 à 55 psig (398 à 487 kPa) à 21°C.

4. Produit de consommation comprenant une composition de pulvérisation cosmétique selon l'une quelconque des revendications précédentes et un dispositif pour produire une pulvérisation à partir de celui-ci.

5. Produit de consommation selon la revendication 4, dans lequel le dispositif de pulvérisation est capable de contenir et de distribuer une composition maintenue à une pression de 43 à 55 psig (de 398 à 487 kPa) à 21°C.

6. Produit de consommation selon la revendication 4 ou 5, dans lequel le dispositif de pulvérisation est capable de produire une pulvérisation de taille de gouttelettes D[4,3] de 10 à 50 microns lorsqu'il est utilisé pour pulvériser la composition à une température de 21°C.

7. Procédé de traitement cosmétique comprenant la pulvérisation d'une composition cosmétique selon l'une quelconque des revendications 1 à 3 sur la surface de la peau d'être humain.
